# EUROPEAN PATENT SPECIFICATION

(11) **EP 1 820 525 B1**
(45) Date of publication and mention of the grant of the patent: **08.04.2009**
(21) Application number: 07250629.8
(22) Date of filing: 15.02.2007
(51) Int. Cl.: A61M 5/158, A61M 25/06

(54) **Low profile infusion set**
Infusionsset mit niedrigem Profil
Perfuseur à faible profil

(30) Priority: 16.02.2006 US 355780
(43) Date of publication of application: 22.08.2007
(73) Proprietor: Animas Corporation, West Chester, PA 19380 (US)
(72) Inventor: Wojcik, Steven E., Shoreline, WA 98177 (US)
(74) Representative: Tunstall, Christopher Stephen

(56) References cited:
- WO-A-02/053220
- WO-A-20/06015507
- WO-A-20/06015600
- US-A1- 2002 173 769
- US-A1- 2004 158 207

## Description

### FIELD OF THE INVENTION

The present invention relates generally to an infusion set, and more particularly to a low profile infusion set used for intermittent or continuous delivery of medication, such as insulin to a patient.

### BACKGROUND OF THE INVENTION

Patients who receive intermittent or continuous doses of medication, such as insulin, via subcutaneous injection, often have an infusion set affixed to their skin in a convenient location. Keeping an infusion set fixed in place is discreet, and reduces the need for repeatedly puncturing the skin with a needle, thereby reducing the risk of infection as well as reducing the formation of scar tissue. The infusion set typically includes a housing supporting a tubular cannula with a removable injection needle at one end for penetrating the skin, and a septum at the other end for receiving a needle attached to a supply tube from a medicinal source, e.g., an insulin pump. One well-known conventional infusion set is a "straight set", in which the cannula and injection needle are inserted in an orientation substantially normal to the skin. The straight set requires a relatively short injection needle, which is less intimidating to some patients, and is relatively easy to insert through the skin. But, because the cannula and injection needle are supported to be oriented normal to the skin, the housing must be upright, conspicuous, and relatively bulky, and furthermore, the cannula, rigidly attached to a bottom of the housing can be subject to kinking and occlusion.

Another known infusion set is a low profile angled set, in which the cannula and injection needle are supported in the housing to be oriented at an acute angle with respect to the skin. The housing of the low profile angled set is less bulky and is much more discreet than the housing of the straight set. However, because of the angled insertion, a much longer injection needle is required, and the longer needle is more intimidating and more difficult to insert, and is subject to inadvertent bending.

Additional problems exist with both the conventional straight and angled sets. For example, a relatively long portion of cannula tubing is left exposed. A view of the injection site is often obscured. Adhesive mounting pads used on the sets can be awkward to use, often prematurely contacting the skin, causing wrinkling of the adhesive pad. Moreover, the needle or the cannula often touch non-sterile tissue or clothing prior to insertion, which increases the risk of infection.

It is desirable, therefore, to provide a low profile, compact infusion set with a relatively short needle, which is easy to position and insert, is easy to connect to the medical source, which does not obscure the patient's view of the injection site, is low and discreet, and in which the cannula is protected from kinking and occlusion.

US 2002/0173769 describes a septum housing rotatably attached to the cannula assembly by a pivot or hinge and a flexible tube attached between septum or septum housing and the cannula using a suitable adhesive.

US 2004/0158207 describes a swivel rotationally mounted in a foundation body which can be moved to a position in which the opening of the swivel points upwards. In this position a cannula can be inserted with the aid of a guiding needle. The guiding needle can be removed and the swivel can then be rotated on its side.

WO 02/053220 describes a delivery tube that can be attached to a rotating member which can then be rotated down to a position along to and adjacent the skin of the patient.

### SUMMARY OF THE INVENTION

Accordingly, the present invention is directed to an infusion set that mitigates or substantially obviates one or more of the shortcomings caused by the limitations and disadvantages of the related art.

The features and advantages of the invention will be set forth in the description which follows, and in part will be apparent from the description, or may be learned by practice of the invention. The advantages of the invention will be realized and attained by the apparatus, and the method of practicing the invention, particularly pointed out in the written description and claims below, as well as in the attached drawings.

In accordance with the present invention, there is provided an infusion set as defined in claim 1.

Further advantages are achieved by the embodiments indicated by the dependent claims.

It is to be understood that both the above general description and the following detailed description are exemplary and explanatory, and are intended to explain the principles of the claimed invention. The accompanying drawings are included to provide a further understanding of the invention and are incorporated and constitute part of the specification, illustrating presently preferred embodiments of the invention.

### BRIEF DESCRIPTION OF THE DRAWINGS

Fig. 1 is a top perspective view of an infusion set in accordance with a first exemplary embodiment of the invention;
Fig. 2 is a side cross-sectional view of along the line 2-2 Fig. 1;
Fig. 3 is a top perspective view of the insertion set of Fig. 1 with the septum housing portion shown separated from the cannula housing portion;
Fig. 4 is an exploded view of the septum housing portion of the first exemplary embodiment of the invention;
Fig. 5 is a side cross-sectional view of the inserted insertion set showing subcutaneous insertion of the cannula and a kink-free bend in the cannula;
Fig. 6 is a perspective view of an exemplary insertion needle assembly useable with the insertion set of Fig. 1;
Fig. 7 is a top perspective view of the exemplary insertion needle assembly of Fig. 6 attached to the exemplary infusion set of Fig. 1;
Fig. 8 is a side cross-sectional view along the line 8-8 in Fig. 7;
Fig. 9 is a front elevation view of the assembly of Fig. 7 with a needle cover attached thereto;
Fig. 10 is a front perspective view of the assembly of Fig. 7 depicting arms of the insertion needle assembly holding side portions of the adhesive pad and paper backing portions folded back to expose adhesive in the center section of the adhesive pad;
Fig. 11 is a front perspective view of the insertion needle assembly of Fig. 6 with a protective cover in place over the insertion needle ready for disposal;
FIG. 12 is a top perspective view of an insertion guide housing supporting the insertion needle assembly of FIG. 6;
FIG. 13 is a side view of the assembly shown in FIG. 12;
FIG. 14 is a top perspective view, similar to FIG. 12, showing the infusion insertion set of Fig. 1 attached to the insertion needle assembly; and
FIG 15 is a top perspective view, similar to FIG. 14, showing depression of the insertion needle assembly.
Fig. 16 is a bottom perspective view of an exemplary needle hub assembly useable with the infusion set of Fig. 1;
Fig. 17 is a top view of the needle hub assembly of Fig. 16 about to be removably attached to the infusion set of Fig. 1, the septum housing portion being in the second position relative to the cannula housing portion;
Fig. 18 is a top perspective view, similar to Fig. 17, showing attachment of the needle hub assembly to the infusion set of Fig. 1;
Fig. 19 is a side cross-sectional view, similar to Fig. 5, illustrating the needle hub assembly attached to the infusion set of Fig. 1; and
Fig. 20 is a top perspective view, similar to Fig. 18, showing the complete tube extending from the needle hub assembly to a fitting for attachment to an external infusion pump.

### DETAILED DESCRIPTION OF THE INVENTION

Although the invention is illustrated and described herein with reference to specific embodiments, the invention is not intended to be limited to the details shown. Rather, various modifications may be made in the details within the scope and range of equivalents of the claims and without departing from the invention.

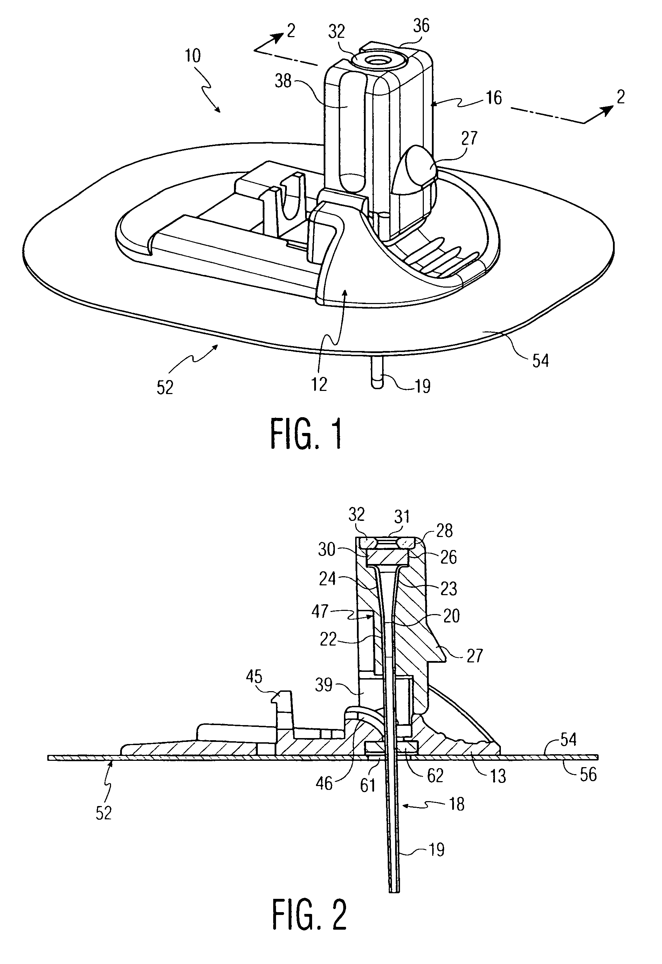

Referring to Figs. 1-5, infusion set 10 that is a first exemplary embodiment of the present invention is shown. As broadly embodied herein, infusion set 10 includes a multiple-part housing, including first or cannula housing portion 12 and second or septum housing portion 16 pivotably attached thereto. Cannula housing 12 includes base surface 13 configured to provide a stable base for insertion set 10.

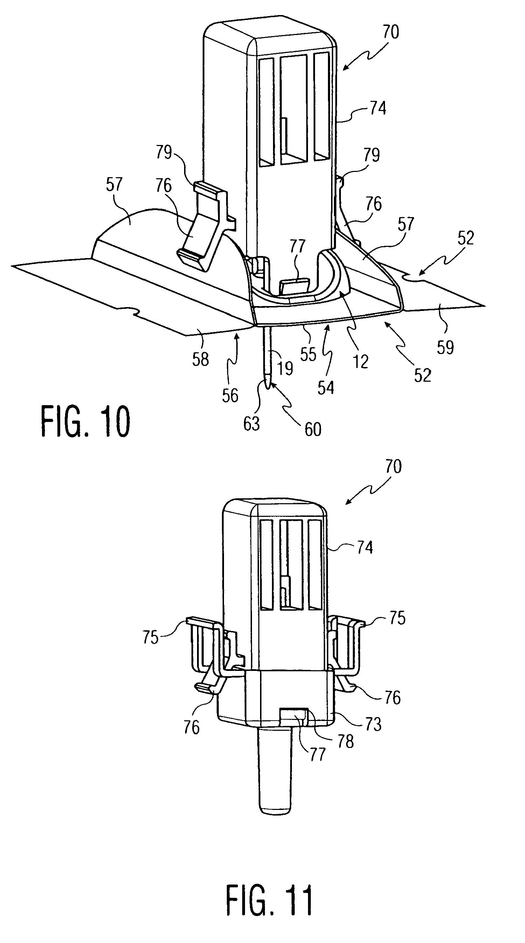

Cannula housing portion 12 is removably attachable to the skin surface 14 of a user via adhesive assembly 52. Adhesive assembly 52 is attached to cannula housing base surface 13 to removably adhere cannula housing portion 12 to skin surface 14. Desirably, adhesive assembly 52 includes sticky adhesive pad 54, which is covered prior to attachment to the skin by a removable paper backing 56. As shown in Fig. 10, pad 54 includes a central portion 55 and two side portions 57. Paper backing 56 includes at least two separately removable paper backing portions 58 and 59. Use of adhesive assembly 52 will be described in more detail hereinafter. Referring again to Figs. 2-4, adhesive assembly 52 has an aperture 61 defined therethrough for passage of distal end 19 of cannula 18. An annular seal 62 is provided in cannula housing portion 12 proximate aperture 61 to seal aperture 61, and shield the interior of cannula housing portion 12 and the injection site from contamination. Alternatively, annular seal 62 may be omitted and the diameter of the proximate aperture 61 through cannula housing 12 may be made just slightly larger than the outer diameter of cannula 19.

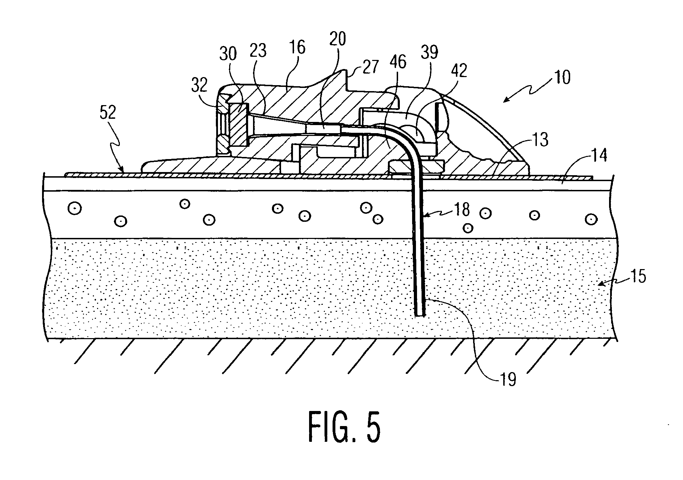

Septum housing portion 16 includes a series of collinear bores 22, 24, 26, and 28, of increasing diameters. Bore 22 has the smallest diameter and extends from a forward end of septum housing portion 16 to bore 24. Bore 24 tapers from bore 24 to bore 26. Bore 26 in turn extends to bore 28. Bore 28 extends to the end of the septum housing portion 16, and has the widest diameter. Bore 24 and a portion of bore 22 are configured to receive ferrule 23. Ferrule 23 has a tapered portion supported in a bore 24 and a cylindrical portion that extends into bore 22. Cannula 18 has a proximal end 20 that is sealingly attached to the cylindrical portion of ferrule 23. Cannula 18 extends out of bore 22 and terminates in distal end 19. As seen in Fig. 2, with septum housing portion 16 connected to cannula housing portion 12, cannula distal end 19 extends through cannula housing portion 12. Cannula 18 desirably is made of medical-grade fluorinated ethylene propylene (FEP) Teflon^{®} or polyurethane, but may be made from any suitable material. As explained in more detail hereinafter, cannula 18 is sized to accept a removable insertion needle to facilitate insertion of distal end 19 of cannula 18 through the user's skin surface 14 and into subcutaneous tissue 15 beneath skin surface 14. (See Fig. 5).

Bore 26 is configured to receive self-sealing elastomeric septum 30 therein. Septum 30 may a round shape as shown, but may be elliptical, rectangular, or any other suitable shape. A stop ring 32 is press fit or the like into bore 28 to retain septum 30 in bore 26. Stop ring 32 has a through bore 31 to permit passage of a needle or the like. Septum 30 seals between stop ring 32 and ferrule 23 to prevent entry of contaminants into cannula 18, however, septum 30 allows passage of a needle or the like. The invention is not limited to the specific configuration of the cannula 18, ferrule 23, septum 30 and stop ring 32 illustrated herein. Other configurations of supporting cannula 18 extending from septum housing portion 16 may also be used.

Septum housing portion 16 includes vertical alignment grooves 36 and 38 defined in the sides thereof, and a pair of depending legs 39 and 40. Each of the depending legs 39 and 40, includes a respective aperture 42 and 44. Apertures 42 and 44 are sized to snap-fit over corresponding pins 35, 37, respectively, projecting from cannula housing portion 12. Engagement of pins 35, 37 and apertures 42, 44 defines a hinged connection for pivotal movement of septum housing portion 16 relative to cannula housing portion 12. Alternately, pins can be provided on septum housing portion 16 with corresponding apertures in cannula housing portion 12. Other forms of pivotal mounting of septum housing 16 relative to cannula housing portion 12 may also be utilized.

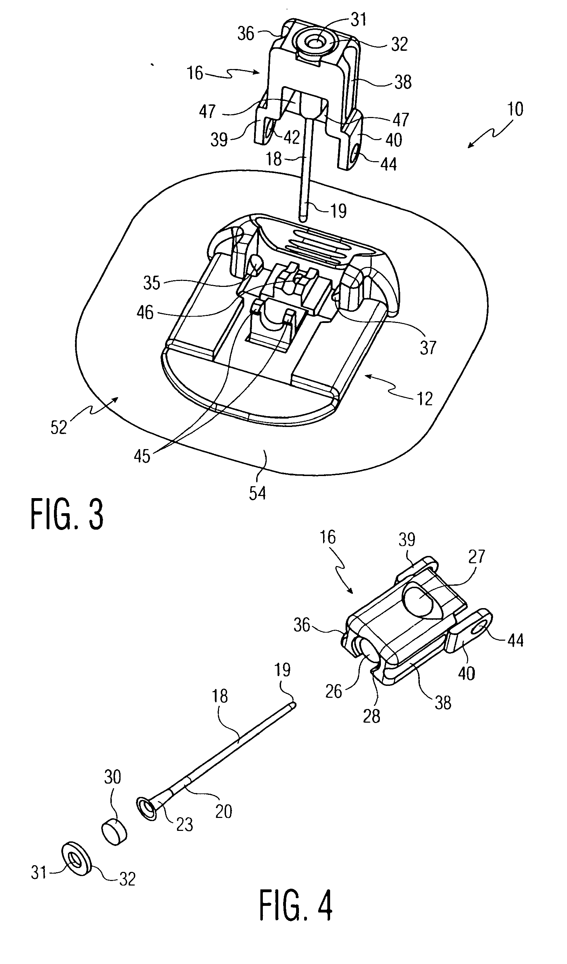

Septum housing portion 16 is configured to pivot with respect to cannula housing portion 12 between a first position as shown in Figs. 1 and 2 and a second position as shown in Fig. 5. In the first position, the axis of septum housing portion 16 extends substantially perpendicular to cannula housing portion base 13, and thereby, substantially normal to skin surface 14 to facilitate a straight insertion. After insertion of cannula 18, septum housing portion 16 is pivoted to the second position where the axis of septum housing portion 16 is substantially parallel to skin surface 14. In the second position, insertion set 10 provides a low profile relative to skin surface 14. As shown in Figs. 2 and 3, a pair of flexible latching arms 45 extend from cannula housing portion 12 and are configured to mate with latching depressions 47 on septum housing portion 16 to lock septum housing portion 16 in place relative to the cannula housing portion 12 in the second position.

The point of pivotal attachment between septum housing portion 16 and cannula housing portion 12 is selected according to a geometrical calculation, such that when septum housing portion 16 pivots relative to cannula housing portion 12, cannula 18 is neither pulled nor pushed. In this way, cannula 18 will not be repositioned with respect to skin surface 14, and will not be inadvertently pulled out of the skin when septum housing portion 16 is pivoted. A geometric calculation of a pivot offset for an axis of rotation by vertical cannula 18 (cannula 18 is vertical when the septum housing portion 16 is in the first position), is shown below:

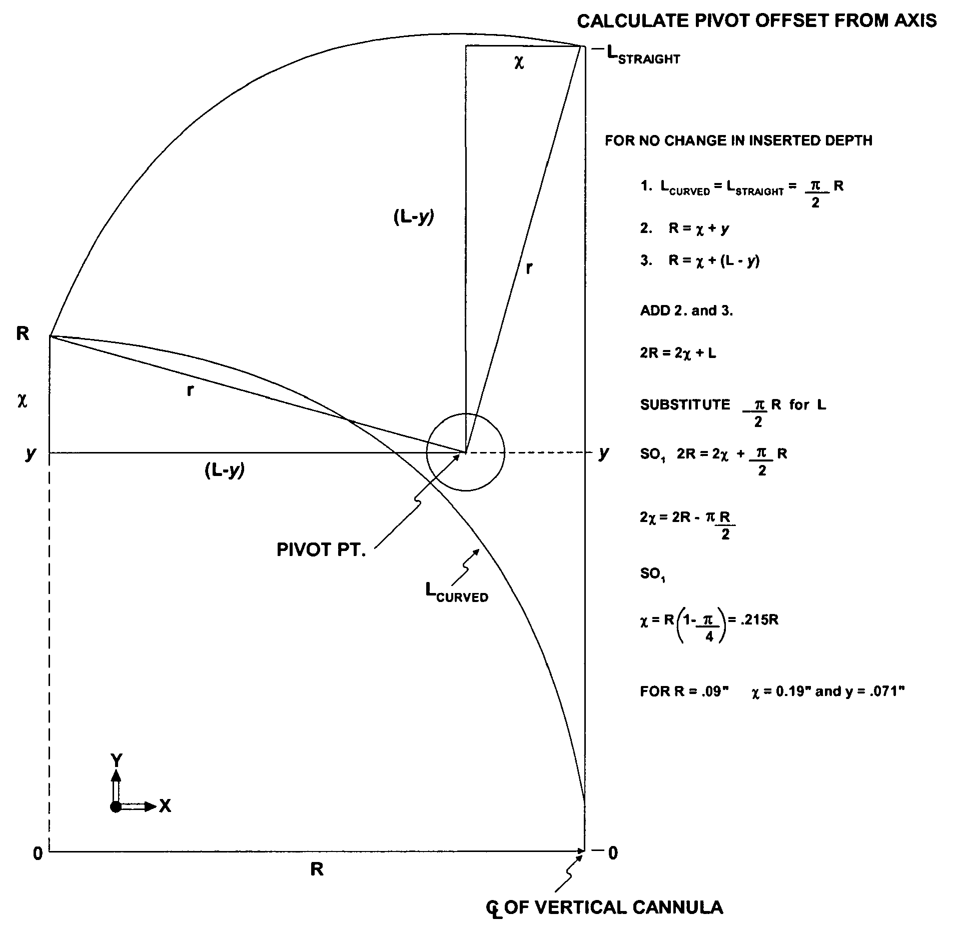

To further protect cannula 18, cannula housing portion 12 further includes a cannula guide or curved mandrel 46, positioned to support an intermediate portion of cannula 18 proximate skin surface 14. Cannula guide 46 provides an arcuate path for cannula 18. As septum housing portion 16 is pivoted to the second position, cannula guide 46 supports the intermediate portion of cannula 18 along a gradual curve. The arcuate path is approximately a fraction of a 90° bend, with a radius in the range of approximately 1-4 mm and desirably about 2.25 mm. This slight curve prevents kinking of cannula 18.

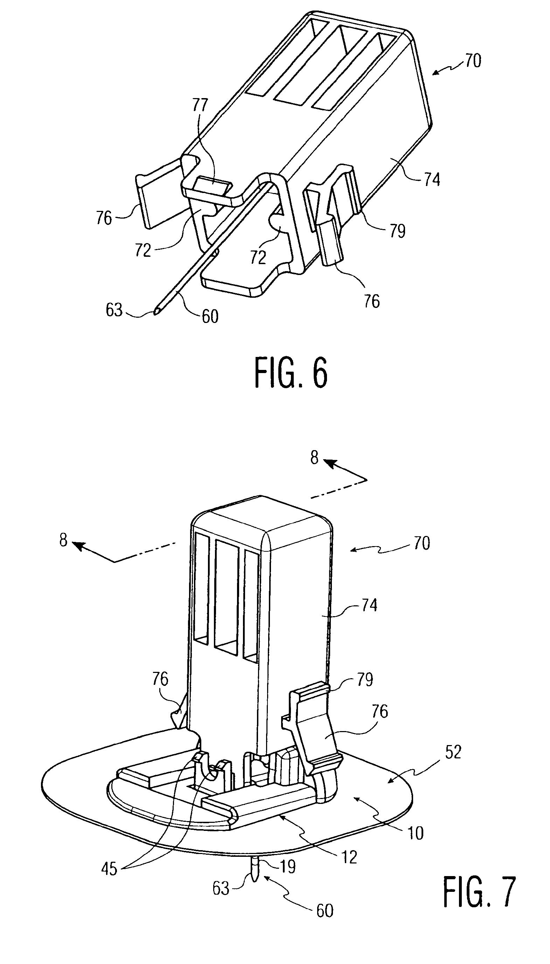

Various insertion needles can be removably inserted through the cannula 18 to insert distal end 19 of cannula 18 into the skin surface 14. A first embodiment of an insertion needle assembly 70 will be described reference to Figs. 6-11. The invention is not limited to insertion needle assembly 70 and the insertion set 11 may be used with various insertion needles.

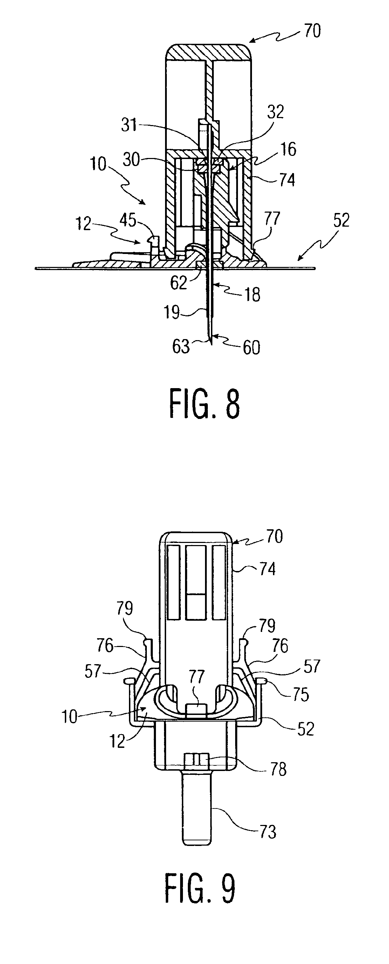

Insertion needle assembly 70 includes a generally hollow housing 74 configured to removably attach to the septum housing portion 16. Removable insertion needle 60 extends from the interior of housing 74 of insertion needle assembly 70. Insertion needle 60 can have various configurations, including but not limited to, a standard beveled needle or a trocar having a sharp tip 63. Internal guide rails 72 on housing 74 are configured to fit slidably into vertical alignment grooves 36, 38 on the sides of septum housing portion 16. Interaction between guide rails 72 and alignment grooves 36, 38 aligns the needle 60 with stop ring throughbore 31 such that insertion needle 60 penetrates septum 30 and passes through cannula 18 out cannula distal end 19 as shown in Fig. 8. Friction between insertion needle 60 and septum 30 and/or cannula 18 is generally high enough to hold insertion needle assembly 70 to insertion set 10 without an additional locking mechanism. A lock mechanism can be provided if desired. Guide rails 72 and alignment guides 36, 38 prevent insertion needle assembly 70 from rotating relative to septum housing portion 16.

Referring to Fig. 9, infusion set 10 may be supplied prepackaged with insertion needle assembly 70 attached thereto. To protect users and maintain the sterility of the prepackaged assembly, cover 73 may be positioned over needle 60 and attached to insertion needle assembly 70. Cover 73 includes extension brackets 75 configured to removably engage extension arms 76 extending from the sides of insertion needle assembly housing 74. The extension brackets 75 also act to fold up side portions 57 of attachment assembly 52, thereby reducing the shipping size of the prepackaged assembly and protecting attachment assembly 52.

To prepare infusion set 10 for use, cover 73 is removed from insertion needle assembly 70, thereby exposing cannula 18 and insertion needle 60. Upon removal of cover 73, extension arms 76 of insertion needle assembly 70 continue to maintain the side portions 57 of the adhesive pad 54 from drooping down as shown in Fig. 10. Prior to insertion, the two halves 58, 59 of backing paper 56 are folded away from center portion 55 of adhesive pad 54 to expose the adhesive surface. Backing paper 56 remains attached to side portions 57 of pad 54 which remain retained by extension arms 76. This allows a wide mounting pad 54 to be used without the side portion 57 from dropping and contacting the skin, as often occurs with prior art devices.

To insert insertion set 10, a user holds insertion needle assembly housing 74 in one hand while pinching a fold of skin with the other hand. Septum housing portion 16, and thereby insertion needle 60, are perpendicular to skin surface 14. Insertion needle assembly housing 74 is pressed straight downward by the user against the user's skin to thereby insert insertion needle 60 and distal end 19 of cannula 18 through skin surface 14 in an orientation substantially normal to the skin surface 14. The force required to insert needle 60 is generally less than 0.5 pounds, and needle 60 desirably is inserted into the skin using only finger pressure, with the speed of insertion being controlled by the user.

Needle 60 is inserted until center portion 55 of adhesive pad 54 contacts skin surface 14. Side portions 57 of pad 54 are applied to skin surface 54 by pulling the halves 58, 59 of backing paper 56 parallel to skin surface 14 while pressing side portions 57 against skin surface 14. Needle 60 is removed by drawing insertion needle assembly 70 away from insertion set 10. Cover 73 can then be positioned over needle 60, as illustrated in Fig. 11, for disposal of insertion needle assembly 70. To maintain cover 73 on insertion needle assembly 70, protrusions 77 (shown in Figs. 8-15) extending from insertion needle assembly housing 74 engage notches 78 in cover 73 (shown in Fig. 11). Once needle 60 is removed, the user pivots septum housing portion 16 down to the second position, and locks it in place as shown in Fig. 5. By engaging 45 into 47, as explained above, curved mandrel 46 prevents kinking of cannula 18. Insertion set 10 is ready for connection to a needle hub assembly 90 as will be described hereinafter.

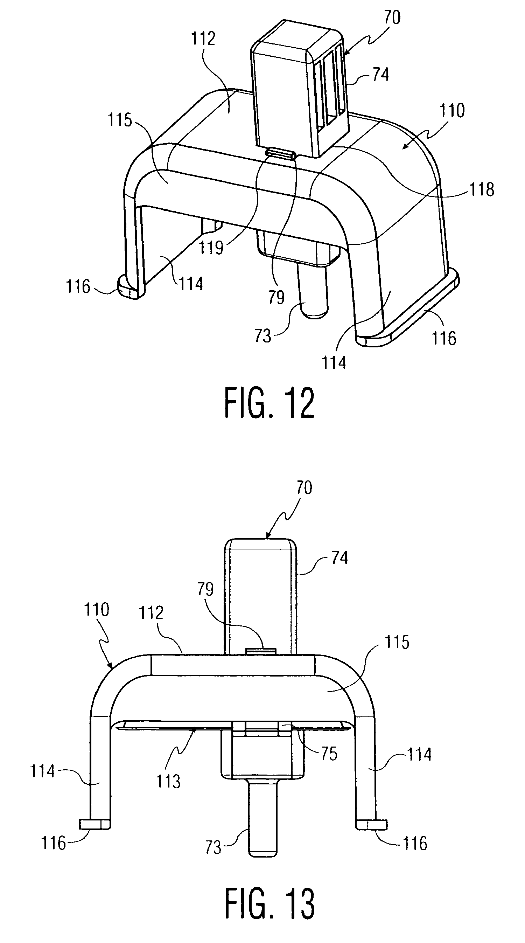

To assist in the insertion process, disposable insertion guide housing 110, as illustrated in Figs. 12-15, may be utilized with insertion needle assembly 70. Insertion guide housing 110 has a generally U-shaped configuration with opposed legs 114 extending downwardly from top surface 112. Each leg 114 terminates in a contact foot 116. Insertion guide housing 110 is configured such that top surface 112 is held generally parallel to skin surface 14 by contact feet 116. Finger grasp sides 115 extend between legs 114 along each side of guide housing 110.

Opening 118 is defined in top surface 112 and has a cross-sectional shape that generally complements the shape of insertion needle assembly housing 74 such that the preassembled insertion set 10 and insertion needle assembly 70, as shown in Fig. 9, may be positioned into insertion guide housing 110. Opening 118 includes a pair of opposed notches 119 configured to receive upper projections 79 extending from the insertion needle assembly extension arms 76 to retain insertion needle assembly 70 extending through insertion guide housing top surface 112, as shown in Figs. 12 and 13. The assembly may be preassembled and ready for use as illustrated in Figs. 12 and 13.

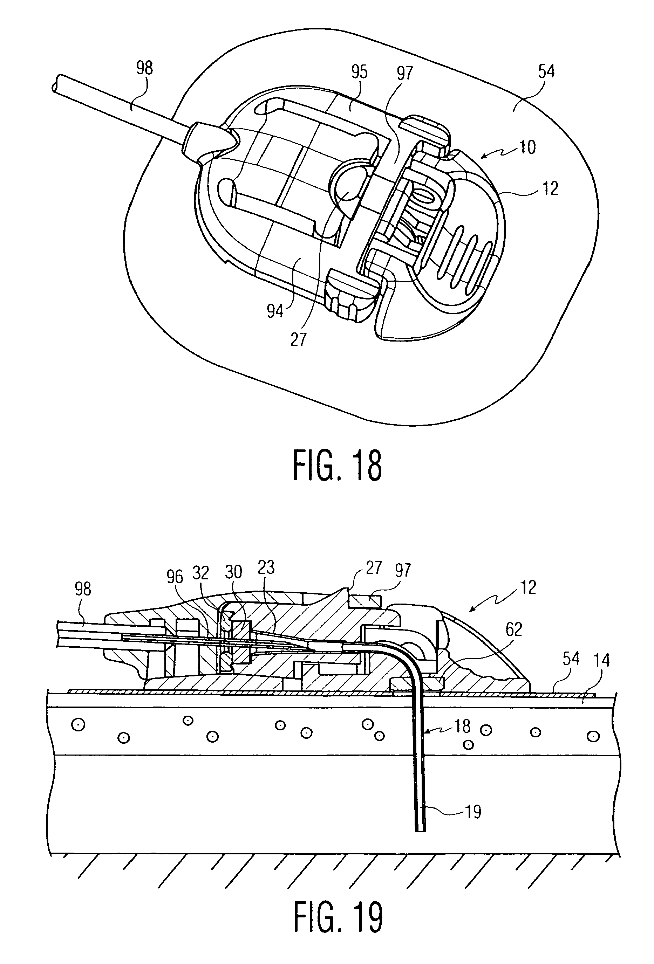

Insertion guide housing 110 has generally open sides 113 which permit flaps of the backing paper 56 on adhesive pad 54 to be folded up parallel to the cannula housing portion bottom while adhesive pad 54 is being adhered to the skin, while the cannula housing portion 12 is mounted in the insertion guide housing 110, as shown in Fig. 18.

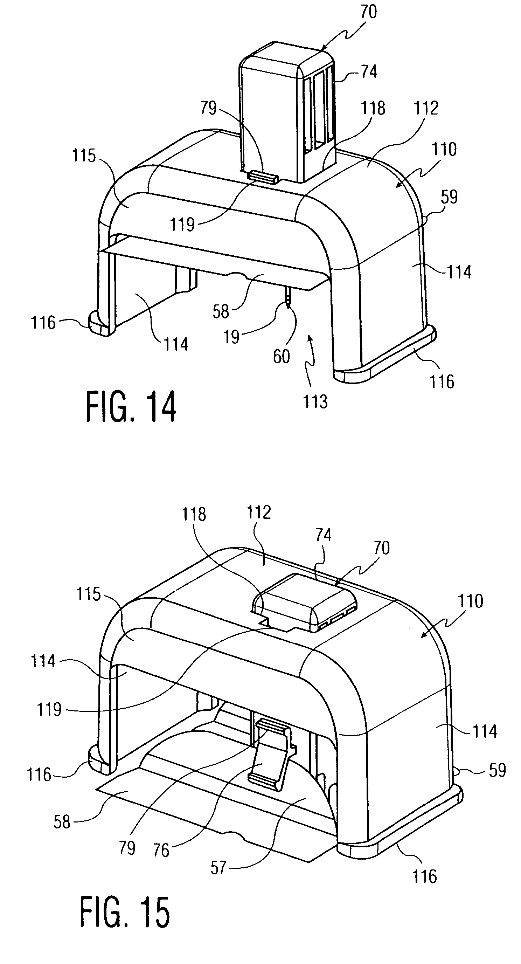

To operate the system, the user picks up the insertion guide housing 110 with one hand using the opposed finger grasp sides 115. As supplied, insertion needle 60 extends through cannula 18, with adhesive pad side portions 57 tucked under the arms 76 of the insertion needle assembly 70, similar to that shown in Figs. 9 and 10. The user removes needle cover 73 and folds back both portions 58 and 59 of backing paper 56 to expose central portion 55 of pad 54, as shown in Fig. 14. Backing paper portions 58 and 59 may be folded up along the finger grasp sides 115 and held out of the way by the user. The user presses feet 116 of insertion guide housing 110 against the skin at the injection site. The user then pushes downward on the top of housing 74 of insertion needle assembly 70 with a light finger force of about 0.1-0.5 lbf. Projections 79 flex and insertion needle assembly 70 pushes needle 60 along with insertion set 10 downward toward skin surface 14. Needle 60 is inserted through the skin surface 14 and cannula distal end 19 moves through aperture 61 to penetrate the skin surface 14 subcutaneously in an orientation substantially normal to skin surface 14. Needle penetration of the skin with finger pressure typically is less painful than spring-loaded needle penetration. The user then pulls away the folded backing paper 56 on each side, applying the side portions of the adhesive pad to the skin without wrinkling pad 54. The user then removes insertion guide housing 110 and insertion needle assembly 70 from septum housing portion 16, thereby withdrawing insertion needle 60 while leaving cannula 18 in place. Septum housing portion 16 is pivoted and locked in the second position illustrated in Fig. 5. Protective cover 73 is placed over needle 60 and insertion guide housing 110 and insertion needle assembly 70 can be discarded.

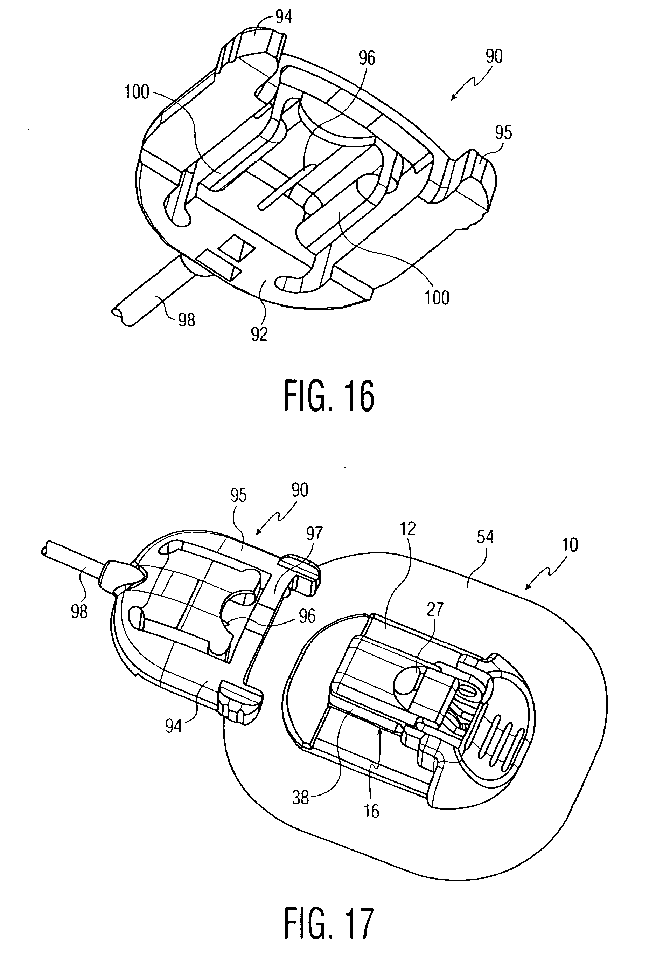

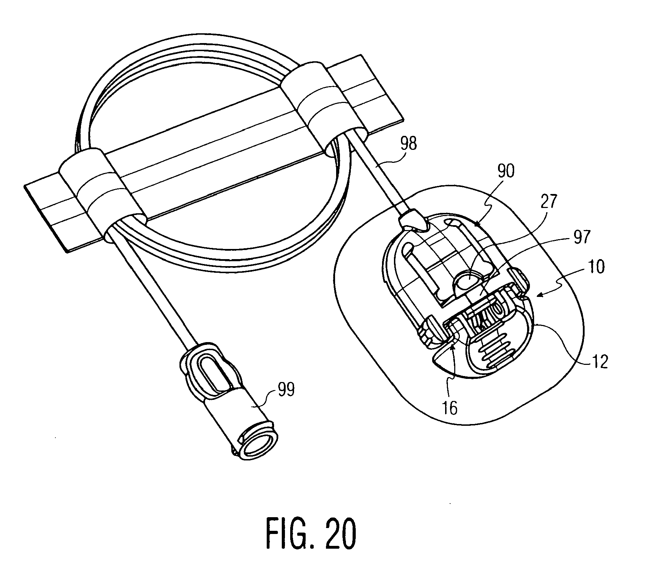

Once insertion set 10 is positioned on the user, in one of the manners above or any other suitable manner, insertion set 10 is ready for connection to an infusion source, for example, an infusion pump, through a needle hub assembly. A first exemplary embodiment of a needle hub assembly 90 useable with insertion set 10 is illustrated in Figs. 16-20. Needle hub assembly 90 is configured to be removably attached to insertion set 10.

Needle hub assembly 90 includes a substantially flat housing 92, resilient band 97, flexible arms 94 and 95, and needle 96. Needle hub assembly 90 also includes guide rails 100 extending along a lower surface of housing 92, inward of flexible arms 94 and 95. Guide rails 100 are configured to align with and slide into grooves 36 and 38 in the sides of septum housing 16. Needle 96 projects between guide rails 100 below an upper surface of housing 92 such that needle 96 is substantially enclosed therein and the user is protected from inadvertent needle sticks when needle hub assembly 90 is disconnected from septum housing portion 16. Sliding of guide rails 100 into respective grooves 36 and 38 guides needle 96 through stop ring 32 and septum 30 such that needle 96 terminates in ferrule 23 as shown in Fig. 19.

To retain needle hub assembly 90 in engagement with septum housing portion 16, resilient band 97 extends between flexible arms 94 and 95 and is configured to flex over and be retained by a retaining bump 27 on septum housing portion 16 as shown in Fig. 18. Retaining bump 27 desirably has a ramped profile when viewed from the side (see Fig. 2) such that resilient band 99 slides freely over retaining bump 27 as needle housing assembly 90 is connected to septum housing portion 16. Resilient band 99 may make an audible click to give the user positive acknowledgment that needle hub assembly 90 and septum housing portion 16 are locked together. To remove needle hub assembly 90, arms 94 and 95 are squeezed together such that resilient band 99 flexes upward to a central height greater than the height of retaining bump 27. Needle hub assembly 90 is slid off of septum housing portion 16, thereby removing needle 96 from septum 30. Other types of engaging and locking assemblies may also be used. For example, a set of complementary barbs and notches (not shown) can be provided on opposing surfaces of needle hub assembly 90 and septum housing portion 16. Other assemblies may also be utilized.

Flexible tube 98 is attached to needle 96 and projects from a rear end of needle hub assembly 90. Tube 98 can extend directly to a medication source, such as an insulin pump, or to an appropriate fitting 99, such as a Luer fitting, which can be connected to an external infusion pump (not shown) or another medicine source. Operation of the medication source supplies medicine through tube 98, through needle 96 and through cannula 18 to deliver the of medicine to the user.

It will be apparent to those skilled in the art that various modifications and variations can be made in the infusion set of the present invention, without departing from the spirit or scope of the invention. Thus, the present invention covers modification and variations of the invention, provided they fall within the scope of the claims, and their equivalents.

## Claims

1. An infusion set (10) comprising:
a cannula housing portion (12) having a base surface (13);
a septum housing portion (16) adapted to be pivotally attached to said cannula housing portion, said septum housing portion having an axis and being pivotable between a first position wherein said axis is substantially perpendicular to said base surface and a second position wherein said axis is substantially parallel to said base surface;
**characterized in that**
a cannula (18) is supported in and extends from said septum housing portion, through said cannula housing portion and from the base surface, such that said cannula is substantially parallel to said axis when said septum housing portion is in the first position; and
said septum housing portion is pivotally attached to said cannula housing at a selected pivot point and further wherein the pivot point is selected so that pivoting of said septum housing portion relative to said cannula housing does not affect the position of that portion of the cannula below the base surface.

2. The infusion set according to claim 1, in which the pivot point is selected so that pivoting of said septum housing portion relative to said cannula housing portion neither pulls nor pushes said cannula.

3. The infusion set according to claim 1 or 2 in which the selected pivot point is offset from the axis in a first position of the septum housing portion.

4. The infusion set according to claim 2 or 3 in which the pivot point is selected by geometrical formula.

5. The infusion set according to claim 4 in which the geometrical formula is χ=R(1-π/4) where χ is the distance from said axis in a first position of the septum housing portion and R is radius of curvature of the cannula.

6. The infusion set of any preceding claim wherein said cannula housing portion includes a cannula guide (46) configured to support a portion of said cannula along an arcuate path when said septum housing portion is in the second position.

7. The infusion set of any preceding claim wherein said arcuate path comprises at least a fraction of a 90° turn.

8. The infusion set of any preceding claim wherein a septum is positioned in said septum housing portion to seal a needle opening to said cannula.

9. The infusion set of any preceding claim further comprising an adhesive assembly (52) attached to said base surface; said adhesive assembly comprises an adhesive pad (54) and a removable backing (56); and further wherein portions of said backing are separately removable from said adhesive pad.

10. The infusion set of claim 9 wherein said adhesive pad defines an aperture (61) therein for passage of said cannula (18) therethrough.

11. The infusion set of claim 10 wherein said cannula housing portion includes an annular seal (62) about said cannula (18) adjacent said aperture (61).

12. The infusion set according to any preceding claim further comprising an insertion needle assembly (70) including an insertion needle assembly housing (74) and a needle supported thereby, wherein said insertion needle assembly housing is configured to engage the septum housing portion with said needle (60) axially aligned with said cannula such that said needle is removably positionable through said cannula.

13. The infusion set of claim 12 wherein said infusion set (11) further comprises an adhesive assembly including an adhesive pad and a backing material, said insertion needle assembly further comprising an arm (76) for holding a portion of the backing material during insertion.

14. The infusion set of claim 12 or 13 wherein the insertion needle assembly includes at least one guide rail (72) adjacent the needle and configured to slidably engage a corresponding groove (36, 38) in said septum housing portion.

15. The infusion set of claim 12, 13 or 14 further comprising an insertion guide housing (110) having a u-shaped configuration defining a top surface (112) and a pair of legs (114) depending therefrom, the top surface having an opening (118) having a configuration which complements an external configuration of said insertion needle assembly housing.

16. The infusion set of claim 15 wherein the insertion needle assembly housing includes flexible projections (79) configured to engage the insertion guide housing adjacent the opening to support the infusion set in a position for insertion.

17. The infusion set according to any preceding claim further comprising a needle hub assembly (90) removably attachable to said septum housing portion and connectable to a source of medication; and wherein said needle hub assembly includes an infusion needle (96) positionable within the cannula; and further wherein the needle hub assembly includes at least one guide rail (100) adjacent the infusion needle and configured to slidably engage a corresponding groove (36, 38) in said septum housing portion.

## Patentansprüche

1. Infusionsbesteck (10), dass aufweist:
einen Kanülen-Gehäusebereich (12) mit einer Basisfläche (13);
einen Septum-Gehäusebereich (16), der so ausgelegt ist, dass er an dem Kanülen-Gehäusebereich schwenkbar zu befestigen ist, wobei der Septum-Gehäusebereich eine Achse hat und zwischen einer ersten Position, in der die Achse im Wesentlichen senkrecht zu der Basisfläche ist, und einer zweiten Position, in der die Achse im Wesentlichen parallel zu der Basisfläche ist, schwenkbar ist;
**dadurch gekennzeichnet, dass**
eine Kanüle (18) in dem Septum-Gehäusebereich gehalten wird und sich von diesem durch den Kanülen-Gehäusebereich und von der Basisfläche erstreckt, so dass die Kanüle im Wesentlichen parallel zu der Achse ist, wenn der Septum-Gehäusebereich in der ersten Position ist; und
der Septum-Gehäusebereich schwenkbar an dem Kanülen-Gehäuse an einem gewählten Schwenkpunkt befestigt ist und wobei weiterhin der Schwenkpunkt so ausgewählt ist, dass das Verschwenken des Septum-Gehäusebereiches in Bezug auf das Kanülen-Gehäuse die Position des Teiles der Kanüle unterhalb der Basisfläche nicht beeinflusst.

2. Infusionsbesteck nach Anspruch 1, bei dem der Schwenkpunkt so ausgewählt ist, dass das Verschwenken des Septum-Gehäusebereiches relativ zu dem Kanülen-Gehäusebereich die Kanüle weder zieht noch schiebt.

3. Infusionsbesteck nach Anspruch 1 oder 2, bei dem der ausgewählte Schwenkpunkt in einer ersten Position des Septum-Gehäusebereiches von der Achse versetzt ist.

4. Infusionsbesteck nach Anspruch 2 oder 3, bei dem der Schwenkpunkt durch eine geometrische Formel ausgewählt ist.

5. Infusionsbesteck nach Anspruch 4, bei dem die geometrische Formel χ = R(1-π/4) ist, wobei χ der Abstand von der Achse in einer ersten Position des Septum-Gehäusebereiches ist und R der Krümmungsradius der Kanüle ist.

6. Infusionsbesteck nach einem der vorangehenden Ansprüche, bei dem der Kanülen-Gehäusebereich eine Kanülenführung (46) umfasst, die so gestaltet ist, dass sie einen Teil der Kanüle entlang einem gebogenen Weg unterstützt, wenn der Septum-Gehäusebereich in der zweiten Position ist.

7. Infusionsbesteck nach einem der vorangehenden Ansprüche, bei dem der gebogene Weg wenigsten einen Teil einer Kehre um 90° aufweist.

8. Infusionsbesteck nach einem der vorangehenden Ansprüche, bei dem ein Septum in dem Septum-Gehäusebereich angeordnet ist, um eine Nadelöffnung zu der Kanüle abzudichten.

9. Infusionsbesteck nach einem der vorangehenden Ansprüche, weiter mit einer haftenden Anordnung (52), die an der Basisfläche befestigt ist; wobei die haftende Anordnung ein haftendes Polster (54) und eine entfernbare Rückenlage (56) aufweist; und bei dem weiterhin Teile der Rückenlage einzeln von dem haftenden Polster entfernbar sind.

10. Infusionsbesteck nach Anspruch 9, bei dem das haftende Polster eine Öffnung (61) für den Durchlass der Kanüle (18) durch diese definiert.

11. Infusionsbesteck nach Anspruch 10, bei dem der Kanülen-Gehäusebereich eine ringförmige Dichtung (62) um die Kanüle (18) angrenzend an die Öffnung (61) umfasst.

12. Infusionsbesteck nach einem der vorangehenden Ansprüche, weiter mit einer Einschubnadelanordnung (70), die ein Einschubnadelanordnungsgehäuse (74) und eine Nadel, die durch dieses gehalten wird, umfasst, wobei das Einschubnadelanordnungsgehäuse so gestaltet ist, dass es an dem Septum-Gehäusebereich anliegt, wobei die Nadel (60) axial mit der Kanüle derart ausgerichtet ist, dass die Nadel entfernbar durch die Kanüle positionierbar ist.

13. Infusionsbesteck nach Anspruch 12, wobei das Infusionsbesteck (11) weiter eine haftende Anordnung aufweist, die ein haftendes Polster und ein Rückenlagematerial umfasst, wobei die Einschubnadelanordnung weiter einen Arm (76) zum Halten eines Teiles des Rückenlagematerials während des Einschiebens aufweist.

14. Infusionsbesteck nach Anspruch 12 oder 13, bei dem die Einschubnadelanordnung wenigstens eine Führungsschiene (72) umfasst, die an die Nadel angrenzt und so gestaltet ist, dass sie verschieblich in eine entsprechende Nut (36, 38) in dem Septum-Gehäusebereich greift.

15. Infusionsbesteck nach Anspruch 12, 13 oder 14, weiter mit einem Einschubführungsgehäuse (110) mit einer u-förmigen Gestaltung, die eine obere Fläche (112) und ein Paar Beine (114), die davon abhängen, definiert, wobei die obere Fläche eine Öffnung (118) mit einer Ausgestaltung hat, die komplementär zu einer äußeren Gestaltung des Einschubnadelanordnungsgehäuses ist.

16. Infusionsbesteck nach Anspruch 15, bei dem das Einschubnadelanordnungsgehäuse flexible Vorsprünge (79) umfasst, die so gestaltet sind, dass sie an dem Einschubführungsgehäuse benachbart der Öffnung angreifen, um das Infusionsbesteck in einer Position für das Einschieben zu unterstützen.

17. Infusionsbesteck nach einem der vorangehenden Ansprüche, weiter mit einer Nadelnabenanordnung (90), die entfernbar an dem Septum-Gehäusebereich befestigt ist und mit einer Quelle für Medikamente verbindbar ist; und bei dem die Nadelnabenanordnung eine Infusionsnadel (96) umfasst, die innerhalb der Kanüle positionierbar ist; und bei dem weiter die Nadelnabenanordnung wenigstens eine Führungsschiene (100) umfasst, die an die Infusionsnadel angrenzt und so gestaltet ist, dass sie verschieblich in eine entsprechende Nut (36, 38) in den Septum-Gehäusebereich greift.

## Revendications

1. Perfuseur (10) comprenant :
une partie de logement de canule (12) ayant une surface de base (13) ;
une partie de logement de septum (16) adaptée pour être fixée de manière pivotante à ladite partie de logement de canule, ladite partie de logement de septum ayant un axe et pouvant pivoter entre une première position dans laquelle ledit axe est sensiblement perpendiculaire à ladite surface de base et une seconde position dans laquelle ledit axe est sensiblement parallèle à ladite surface de base ;
**caractérisé en ce que**
une canule (18) est supportée dans ladite partie de logement de septum et s'étend à partir de celle-ci, à travers ladite partie de logement de canule et depuis la surface de base, de telle sorte que ladite canule est sensiblement parallèle audit axe lorsque ladite partie de logement de septum est dans la première position ; et
ladite partie de logement de septum est fixée de façon pivotante audit logement de canule au niveau d'un point de pivotement choisi, le point de pivotement étant choisi en outre de telle sorte que le pivotement de ladite partie de logement de septum par rapport audit logement de canule n'affecte pas la position de la partie de la canule en dessous de la surface de base.

2. Perfuseur selon la revendication 1, dans lequel le point de pivotement est choisi de telle sorte que le pivotement de ladite partie de logement de septum par rapport à ladite partie de logement de canule ne tire jamais sur ladite canule ni ne la pousse.

3. Perfuseur selon la revendication 1 ou 2, dans lequel le point de pivotement choisi est décalé par rapport à l'axe dans une première position de la partie de logement de septum.

4. Perfuseur selon la revendication 2 ou 3, dans lequel le point de pivotement est choisi grâce à la formule géométrique.

5. Perfuseur selon la revendication 4, dans lequel la formule géométrique est χ=R(1-π/4), où χ est la distance par rapport audit axe dans une première position de la partie de logement de septum et R est le rayon de courbure de la canule.

6. Perfuseur selon l'une quelconque des revendications précédentes, dans lequel ladite partie de logement de canule comprend un guide de canule (46) configuré pour supporter une partie de ladite canule le long d'une trajectoire courbe lorsque ladite partie de logement de septum est dans la seconde position.

7. Perfuseur selon l'une quelconque des revendications précédentes, dans lequel ladite trajectoire courbe comprend au moins une fraction d'un tour de 90°.

8. Perfuseur selon l'une quelconque des revendications précédentes, dans lequel un septum est positionné dans ladite partie de logement de septum pour fermer hermétiquement une ouverture d'aiguille sur ladite canule.

9. Perfuseur selon l'une quelconque des revendications précédentes comprenant en outre un ensemble adhésif (52) fixé à ladite surface de base ; dans lequel ledit ensemble adhésif comprend un tampon adhésif (54) et une protection amovible (56) ; et dans lequel en outre des parties de ladite protection sont amovibles séparément dudit tampon adhésif.

10. Perfuseur selon la revendication 9, dans lequel ledit tampon adhésif définit une ouverture (61) à l'intérieur de celui-ci pour le passage de ladite canule (18).

11. Perfuseur selon la revendication 10, dans lequel ladite partie de logement de canule comprend un joint annulaire (62) autour de ladite canule (18) adjacent à ladite ouverture (61).

12. Perfuseur selon l'une quelconque des revendications précédentes, comprenant en outre un ensemble d'aiguille d'insertion (70) contenant un logement d'ensemble d'aiguille d'insertion (74) et une aiguille supportée par celui-ci, dans lequel ledit logement d'ensemble d'aiguille d'insertion est configuré pour mettre en prise la partie de logement de septum avec ladite aiguille (60) en alignement axial avec ladite canule, de telle sorte que ladite aiguille est positionnable de façon amovible à travers ladite canule.

13. Perfuseur selon la revendication 12, dans lequel ledit perfuseur (11) comprend en outre un ensemble adhésif contenant un tampon adhésif et un matériau de protection, ledit ensemble d'aiguille d'insertion comprenant en outre un bras (76) pour maintenir une partie du matériau de protection lors de l'insertion.

14. Perfuseur selon la revendication 12 ou 13, dans lequel l'ensemble d'aiguille d'insertion comprend au moins un rail de guidage (72) adjacent à l'aiguille et configuré pour se mettre en prise de façon coulissante avec une rainure correspondante (36, 38) dans ladite partie de logement de septum.

15. Perfuseur selon la revendication 12, 13 ou 14, comprenant en outre un logement de guide d'insertion (110) ayant une configuration en U définissant une surface supérieure (112) et une paire de pattes (114) partant de celle-ci, la surface supérieure ayant une ouverture (118) ayant une configuration qui complète une configuration externe dudit logement d'ensemble d'aiguille d'insertion.

16. Perfuseur selon la revendication 15, dans lequel le logement d'ensemble d'aiguille d'insertion comprend des saillies flexibles (79) configurées pour se mettre en prise avec le logement de guide d'insertion adjacent à l'ouverture pour supporter le perfuseur dans une position d'insertion.

17. Perfuseur selon l'une quelconque des revendications précédentes, comprenant en outre un ensemble raccord d'aiguille (90) pouvant être fixé de façon amovible à ladite partie de logement de septum et raccordable à une source de médication ; et dans lequel ledit ensemble raccord d'aiguille comprend une aiguille de perfusion (96) positionnable à l'intérieur de la canule ; et dans lequel en outre l'ensemble raccord d'aiguille comprend au moins un rail de guidage (100) adjacent à l'aiguille de perfusion et configuré pour se mettre en prise de façon coulissante avec une rainure correspondante (36, 38) dans ladite partie de logement de septum.
